## Europäisches Patentamt
## European Patent Office
## Office européen des brevets

(19)

(11) Numéro de publication: **0 226 515**
**B1**

(12)

# FASCICULE DE BREVET EUROPEEN

(45) Date de publication du fascicule du brevet:
**16.08.90**

(51) Int. Cl.⁵: **A61B 6/04**

(21) Numéro de dépôt: 86402753.7

(22) Date de dépôt: **10.12.86**

(54) Table d'examen à déplacement longitudinal d'un panneau.

(30) Priorité: **13.12.85 FR 8518531**

(43) Date de publication de la demande:
**24.06.87 Bulletin 87/26**

(45) Mention de la délivrance du brevet:
**16.08.90 Bulletin 90/33**

(84) Etats contractants désignés:
**DE IT NL**

(56) Documents cités:
**EP-A- 0 015 612**
**EP-A- 0 097 086**
**US-A- 4 475 072**

(73) Titulaire: **GENERAL ELECTRIC CGR S.A., 100, rue Camille-Desmoulins, F-92130 Issy les Moulineaux(FR)**

(72) Inventeur: **Jarin, Jean-Pierre, THOMSON-CSF SCPI 19, avenue de Messine, F-75008 Paris(FR)**

(74) Mandataire: **Ballot, Paul Denis Jacques, Cabinet Ballot-Schmit 7, rue le Sueur, F-75116 Paris(FR)**

## Description

La présente invention concerne une table d'examen dont le panneau porte-patient est déplaçable selon son axe longitudinal; l'invention concerne particulièrement un agencement de moyens servant à assurer ce déplacement.

Dans les tables d'examen radiologique, il est courant de déplacer un panneau porte-patient par rapport à un élément fixe, tel que le socle de la table d'examen par exemple. Le déplacement s'effectue généralement sous l'actionnement de moyens moteurs, solidaires du socle, et dont le mouvement de rotation est transformé en un mouvement linéaire transmis au panneau par un système de poulies et de courroies. En général un arbre moteur est mis en rotation sur son axe et entraîne, par l'intermédiaire d'au moins un pignon cranté, une ou des courroies dont les extrémités sont fixées au panneau à déplacer ; les courroies circulent contre différentes poulies et le déplacement des courroies entraîne le déplacement du panneau.

Le panneau est déplacé sur des moyens support tels que par exemple, d'une part un élément mobile le long de l'axe longitudinal, fixé à une extrémité du panneau, et d'autre part un rouleau disposé sous le panneau selon un axe transversal à la longueur du panneau. Ces moyens support peuvent être également constitués uniquement par des rouleaux supports disposés selon des axes transversaux à la longueur du panneau ; au moins deux rouleaux supports étant nécessaires si leur longueur correspond sensiblement à la largeur du panneau. D'autre part, des galets de réaction doivent être disposés sur les côtés longitudinaux du panneau, en réaction à chaque rouleau.

Ces différentes poulies, ces différents rouleaux, ces différents galets de réaction sont tenus par des axes supports solidarisés à la partie fixe de la table, c'est-à-dire au socle. Un des problèmes que posent ces axes supports, par leur nombre, réside en ce que leur répartition judicieuse conduit à conférer au socle une dimension importante, parallèlement à l'axe longitudinal du panneau. Cette dimension importante du socle constitue dans la plupart des cas une gêne, particulièrement dans les tables d'examen à panneaux déplaçables, ou d'une part un accès aisé du patient est nécessaire, et où d'autre part le maximum de place doit être libéré pour placer les différents appareils de radiologie. Une table d'examen du type décrit ci-dessus est connue de US-A 4 475 072.

La multiplication de ces axes supports conduit en outre à augmenter de manière non négligeable le coût de la table d'examen.

La présente invention concerne une table d'examen dont le panneau porte-patient est déplaçable selon son axe longitudinal, et dans laquelle, des moyens qui servent à réaliser ce déplacement, sont disposés selon un agencement nouveau qui permet d'en réduire le nombre et l'encombrement, tout en conservant ses qualités au déplacement du panneau.

L'invention concerne une table d'examen comprenant:

– un panneau à déplacement suivant un axe longitudinal,
– un socle fixe,
– un arbre moteur, fixé audit socle et tournant autour d'un premier axe perpendiculaire à l'axe longitudinal dudit panneau,
– des premiers moyens pour entraîner en rotation ledit arbre moteur,
– au moins un premier rouleau monté sur ledit arbre moteur pour supporter ledit panneau,
– au moins un second rouleau fixé audit socle et tournant autour d'un second axe perpendiculaire à l'axe longitudinal dudit panneau pour supporter ledit panneau à une certaine distance dudit premier axe,
– au moins un troisième rouleau fixé audit socle et tournant autour d'un troisième axe perpendiculaire à l'axe longitudinal dudit panneau, ledit troisième axe étant disposé à proximité du premier axe et ledit troisième rouleau étant en contact avec une surface de roulement dudit panneau de manière à exercer une force de réaction vis-à-vis du premier rouleau,
– au moins un quatrième rouleau fixé audit socle et tournant autour d'un quatrième axe perpendiculaire à l'axe longitudinal dudit panneau, ledit quatrième axe étant disposé à proximité dudit second axe et ledit quatrième rouleau étant en contat avec ladite surface de roulement dudit panneau de manière à exercer une force de réaction vis-à-vis dudit second rouleau,
– des seconds moyens pour convertir le mouvement circulaire de rotation dudit arbre moteur en un mouvement linéaire dudit panneau parallèlement audit axe longitudinal, caractérisé en ce que ledit premier rouleau est monté sur ledit arbre moteur de manière à tourner indépendamment dudit arbre moteur par l'intermédiaire d'un premier roulement, et en ce que ledit troisième rouleau est monté sur un bras monté sur ledit arbre moteur par l'intermédiaire d'un second roulement.

L'invention sera mieux comprise grâce à la description qui suit, faite à titre d'exemple non limitatif, et aux trois figures annexées parmi lesquelles:

– la figure 1 montre par une vue en perspective, la configuration générale d'une table d'examen conforme à l'invention;
– la figure 2 montre la table d'examen par une vue de côté, selon une flèche B présentée à la figure 1, de manière à montrer un panneau d'examen selon sa longueur;
– la figure 3 montre la table d'examen par une vue en coupe transversale selon des flèches A-A montrées à la figure 2 ;
– la figure 4 représente une partie d'un rouleau montrée à la figure 3 dans un encadré.

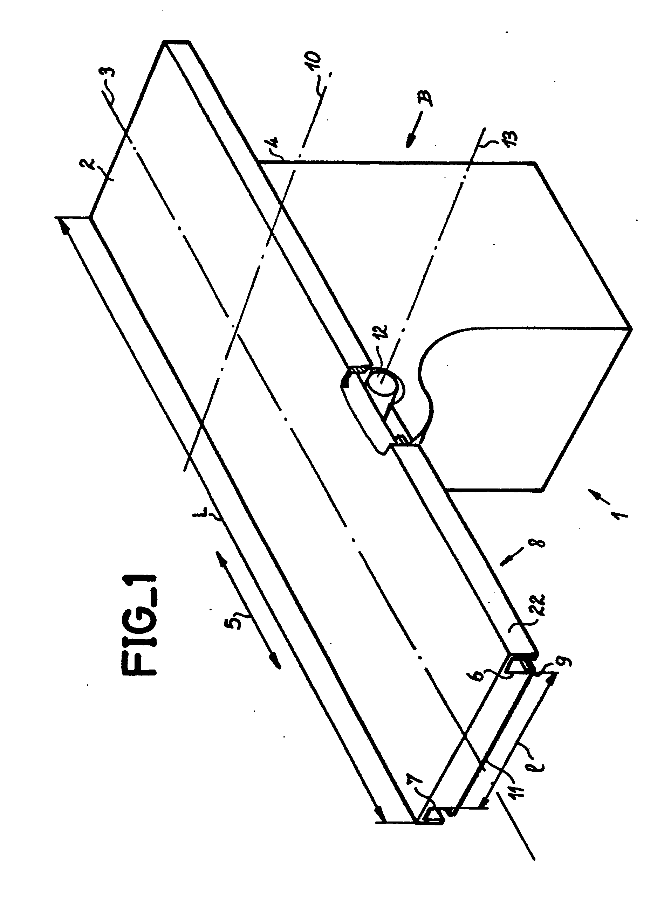

La figure 1 représente par une vue en perspective, la configuration générale d'une table d'examen selon l'invention.

La table d'examen 1 comporte un panneau 2 destiné à supporter un patient. Le panneau 2 est déplaçable selon son axe longitudinal 3, comme montré par la première flèche 5, par rapport à un socle 4, grâce à des moyens de déplacement non représentés sur la figure 1 ; ces moyens de déplacement comportant notamment un arbre moteur disposé selon un premier axe 10 transversal à la longueur du panneau 2.

Dans l'exemple non limitatif décrit, le panneau 2 comporte sur ses premier et second côtés longitudinaux 6,7 une gouttière de protection 8, sous laquelle s'étend selon la longueur L du panneau 2, un bord plat 9 en dépassement par rapport à la largeur l du panneau 2 ; la gouttière 8 comportant un bord 22 courbé vers une face inférieure 11 du panneau 2. Le bord plat 9 est sensiblement dans le plan de la face inférieure 11 du panneau 2, et constitue une surface de roulement comme il est davantage expliqué dans une suite de la description.

Selon une caractéristique de l'invention, des moyens supports du panneau 2 comportent un support formé d'au moins un rouleau (non représenté sur la figure 1), disposé selon le même premier axe 10 transversal au panneau 2 que l'arbre moteur précédemment cité. Dans l'exemple non limitatif décrit, les moyens supports comportent d'autre part au moins un second rouleau 12 disposé selon un second axe 13 parallèle au premier axe 10 ; une extrémité du second rouleau 12 étant montrée sur la figure 1 grâce à une ouverture pratiquée dans le dessin de la gouttière 8 et du bord plat 9.

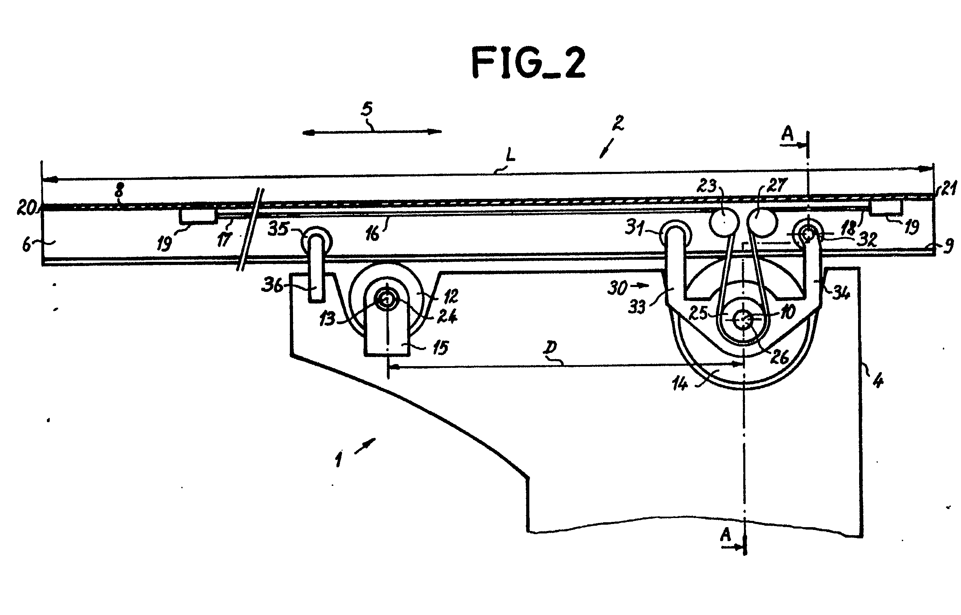

La figure 2 montre la table d'examen par une vue de côté selon la flèche B représentée à la figure 1, de manière à représenter le panneau 2, partiellement, selon sa longueur L et par son premier côté longitudinal 6 ; le bord 22 de la gouttière 8 n'étant pas représenté.

Ainsi qu'il a été mentionné précédemment, le panneau 2 est supporté d'une part par au moins un rouleau 14 disposé selon le premier axe 10, et constitué par exemple d'un rouleau unique ou par plusieurs rouleaux ; le panneau 2 est supporté d'autre part, par au moins un second support constitué par au moins un second rouleau 12. Dans l'exemple non limitatif décrit, le rouleau 12 comporte, perpendiculairement au plan de la figure, une longueur (non représentée) sensiblement égale à la largeur l précédemment mentionnée du panneau 2, mais peut être constitué également selon une pratique courante, par deux rouleaux de longueur plus faible disposés sur le même second axe 13, l'un de ces rouleaux étant par exemple plus près du premier côté longitudinal 6 et l'autre rouleau plus près du second côté longitudinal 7. Le second rouleau 12 est d'un type en lui-même connu, monté de manière classique sur un axe support 24, de manière à être mobile en rotation autour du second axe 13, tout en étant solidarisé à ses extrémités opposées au socle 4 par des bras supports 15.

Dans l'exemple non limitatif de la description, les moyens de déplacement servant à déplacer le panneau 2 selon son axe longitudinal 3, comportent au moins une courroie 16 dont les première et seconde extrémités 17,18 sont fixées, par des moyens de fixation 19 classiques, respectivement du côté d'une première et d'une seconde extrémités 20,21 du panneau 2 ; les fixations 19 étant solidarisées au panneau 2, à la gouttière 8 par exemple. La courroie 16 est disposée dans la partie protégée par la gouttière 8, et, à partir de sa première extrémité 17, elle longe le panneau 2 jusqu'à tourner autour d'une première poulie 23 montée libre en rotation ; après la première poulie 23, la courroie 16 est renvoyée dans une direction sensiblement opposée au plan du panneau 2, jusqu'à tourner autour d'un pignon cranté 25 monté sur l'arbre moteur 26 précédemment mentionné. La courroie 16 retourne ensuite vers le plan du panneau 2 et tourne à nouveau sur une seconde poulie 27 sensiblement alignée avec la première poulie 23 dans le sens de la longueur L du panneau 2. La courroie 16 tourne autour de la deuxième poulie 27 pour s'étendre en direction de la seconde extrémité 21 du panneau 2 où elle est fixée au moyen de fixation 19 ; un montage semblable pouvant être réalisé sur le second côté longitudinal 7 (non représenté) du panneau 2.

En fonctionnement, l'arbre moteur 26 est mis en rotation sur lui-même autour du premier axe transversal 10, grâce à des moyens moteurs classiques (non représentés) disposés par exemple dans le socle 4 ; le pignon cranté 25 étant fixé sur l'arbre moteur 26, il entraîne le déplacement de la courroie 16, qui elle-même entraîne le déplacement du panneau 2 selon son axe longitudinal 3, dans l'un ou l'autre des sens représentés par la première flèche 5, selon le sens de rotation de l'arbre moteur 26.

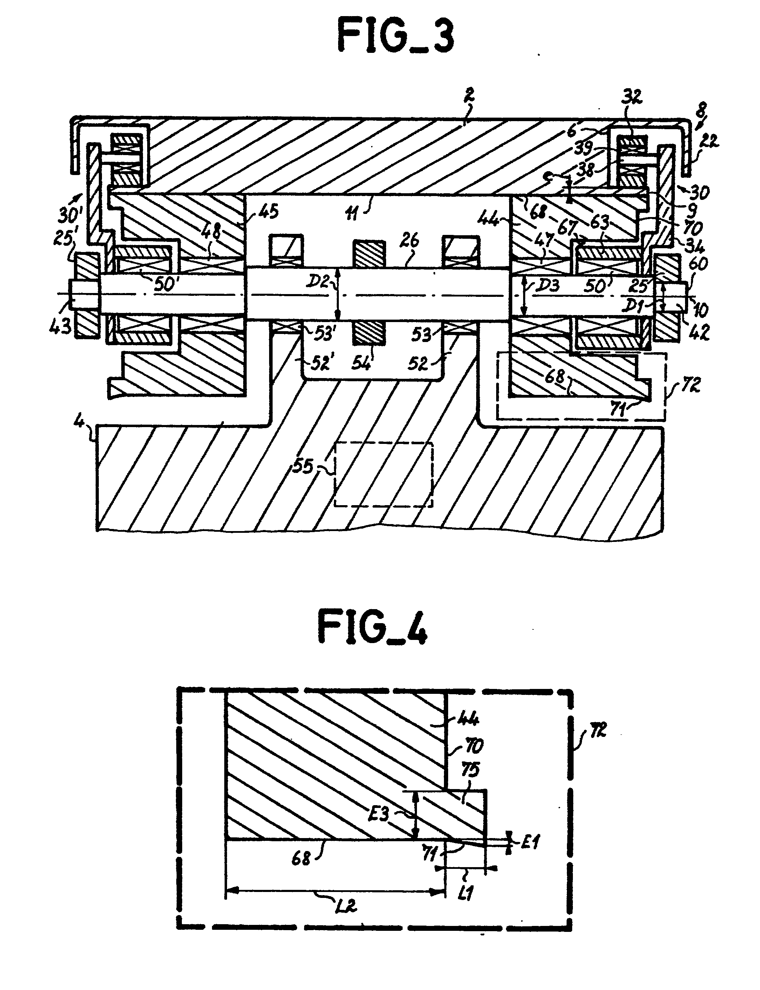

Ainsi qu'il est davantage expliqué dans une partie de la description relative à la figure 3, le premier rouleau 14 supporte le panneau 2 selon le même premier axe 10 transversal que celui selon lequel est disposé l'arbre moteur 26. Le déplacement du panneau 2 en appui sur les rouleaux 12, 14 provoque la rotation de ceux-ci autour de leur axe 13, 10, et pour que la rotation du premier rouleau 14 soit indépendante de la rotation de l'arbre moteur 26, le premier rouleau 14 est monté sur l'arbre moteur 26 par l'intermédiaire d'un moyen de roulement (non représenté sur la figure 2). L'arbre moteur 26 constitue ainsi, outre sa fonction de commande de déplacement du panneau 2, un axe support pour le premier rouleau 14, et pour éventuellement d'autres moyens destinés à conserver une position fixe par rapport au socle 4.

La réaction à la poussée du premier rouleau 14 sur le panneau 2, est exercée par un dispositif de réaction 30 à galets. Dans l'exemple non limitatif décrit, le dispositif de réaction 30 comporte un premier et un second galets 31, 32 disposés de part et d'autre des première et seconde poulies 23,27, et qui sont en appui sur le bord plat 9 sur lequel ils roulent dans le déplacement du panneau 2 ; les premier et second galets 31,32 étant respectivement portés par un premier et un second bras 33, 34.

Dans cette configuration les premier et second galets 31,32 apportent chacun une réaction au premier rouleau 14 dans les meilleures conditions, ces premier et second galets étant disposés de manière symétrique de part et d'autre du rouleau 14, et dans cette configuration, il est intéressant que le dispositif de réaction 30 soit porté par un axe support disposé selon le premier axe 10.

A cette fin, selon une caractéristique de l'invention, le dispositif de réaction 30 est monté sur l'arbre moteur 26 par l'intermédiaire de second moyens de roulement (non représentés) afin de n'être pas dépendant de la rotation de l'arbre moteur 26.

On observe que cet agencement évite l'utilisation d'un axe support supplémentaire pour le premier rouleau 14 et d'un autre axe support supplémentaire pour le dispositif de réaction 30 ; ceci permet notamment de bénéficier d'une distance D entre l'axe 10 du premier rouleau 14 et l'axe 13 du second rouleau 12 aussi faible que possible, tout en tenant compte de ce que la distance D doit avoir une valeur minimum pour que le panneau soit supporté d'une manière correcte ; l'implantation d'un support d'axe supplémentaire pour porter par exemple le premier rouleau 14, aurait conduit à augmenter la dimension du socle 4 parallèlement à l'axe longitudinal 3.

Dans l'exemple non limitatif décrit, un troisième galet 35 est porté par un étrier 36, fixé au socle 4 à proximité du second rouleau 12, afin d'équilibrer la poussée de ce dernier.

La figure 3 montre la table d'examen par une vue en coupe transversale selon des flèches A-A montrées sur la figure 2.

L'arbre moteur 26 est disposé sous le panneau 2 selon le premier axe 10, ses première et seconde extrémités 42, 43 étant en dépassement par rapport au bord plat 9.

Dans l'exemple non limitatif de la description, le premier rouleau 14 est constitué par un premier et un second rouleaux d'extrémité 44,45 portés respectivement par la première et seconde extrémités 42,43 de l'arbre moteur 26, et par l'intermédiaire respectivement d'un premier et d'un second moyens de roulement 47,48. Le premier dispositif de réaction 30 et un second dispositif de réaction 30' sont montés également sur l'arbre moteur 26 respectivement à la première et à la seconde extrémités 42,43 de ce dernier ; les dispositifs de réaction 30,30' étant montés sur l'arbre moteur 26 par l'intermédiaire respectivement d'un troisième et d'un quatrième moyens de roulement 50,50'. Enfin, les extrémités 42,43 proprement dites de l'arbre moteur 26 sont occupées par un premier et un second pignons crantés 25,25', montés de manière à être solidaires de l'arbre moteur 26, et à tourner avec ce dernier ; chaque pignon cranté entraînant le déplacement d'une courroie 16 (non représentée sur la figure 3) comme il a été précédemment expliqué.

L'arbre moteur 26 est solidarisé au socle 4 d'une manière en elle-même connue. Le socle 4 comporte à cet effet un premier et un second montants 52,52' auxquels l'arbre moteur 26 est fixé par l'intermédiaire d'un cinquième et sixième moyens de roulement 53,53' afin de permettre sa rotation. Dans l'exemple non limitatif décrit, l'arbre moteur 26 comporte dans une partie centrale de sa longueur, un troisième pignon cranté 54, par lequel il est mis en rotation sous l'action de moyens moteurs 55, symbolisés dans un rectangle en traits pointillés ; ces moyens moteurs 55 sont par exemple contenus dans le socle 4, et coopèrent avec le troisième pignon cranté 54, par l'intermédiaire d'une courroie par exemple (non représentée), pour la rotation de l'arbre moteur 26.

La description qui suit précise le montage qui vient d'être décrit, en référence uniquement à la première extrémité 42 de l'arbre moteur 26, l'exemple valant également pour la seconde extrémité 43, ces montages étant symétriques.

En partant de la première extrémité 42, on trouve en premier lieu le premier pignon cranté 25 monté sur l'arbre moteur 26, sur une partie extrême 60 de ce dernier ; la partie extrême 60 ayant, dans l'exemple non limitatif décrit, un premier diamètre D1 inférieur au second diamètre D2 que comporte l'arbre moteur 26 dans sa partie centrale. On trouve ensuite en allant vers l'intérieur du panneau 2, le troisième moyen de roulement 50, monté sur l'arbre moteur 26 sur une partie de ce dernier comportant un troisième diamètre D3 compris entre le premier et le second diamètres D1, D2. Le troisième moyen de roulement 50 est solidaire du dispositif de réaction 30, dont la figure 3 montre le second bras 34 portant le second galet 32 dans un plan plus profond que celui de la figure ; le second galet 32 étant solidarisé au bras 34 par l'intermédiaire d'un axe et d'un roulement 38, 39. Le second bras 34 est disposé selon un plan sensiblement vertical, entre un bord extérieur du bord plat 9 et la partie courbée 22 de la gouttière 8 ; le second bras 34 est solidarisé au troisième moyen de roulement 50 par l'intermédiaire d'une pièce 63, le troisième moyen de roulement étant disposé sur une partie de l'arbre moteur 26 située sensiblement en vis à vis du bord plat 9, c'est-à-dire d'avantage vers l'intérieur du panneau 2. On trouve ensuite le premier moyen de roulement 47 par l'intermédiaire duquel le premier rouleau d'extrémité 44 est supporté par l'arbre moteur 26.

Le premier rouleau d'extrémité 44 comporte une cavité centrale 67 ouverte, sur un côté 70 orienté vers la première extrémité 42 de l'arbre moteur 26, de manière à contenir le troisième roulement 50 et la pièce 63 par laquelle ce dernier est fixé au second bras 34 et au premier bras 33 (non représenté sur la figure 3). Ceci permet de conférer au premier rouleau d'extrémité 44 une surface portante 68 qui est en appui sur la face inférieure 11 du panneau 2 et sur le bord plat 9, entre ce dernier et la pièce 63, de manière à supporter le panneau 2 sensiblement sous le premier bord longitudinal 6 où il a sa plus grande rigidité, et sous le bord plat 9 sur lequel s'exerce la réaction du second galet 32.

Le premier rouleau d'extrémité 44 est réalisé en un matériau classique, tel qu'à base de polyuréthane par exemple, lui permettant de présenter une rigidité mécanique suffisante et également une certaine souplesse, notamment pour compenser des éventuelles variations de l'épaisseur $e$ du bord plat 9.

Les variations d'épaisseur $e$ du bord plat 9 quand elles sont dans le sens de l'augmentation, tendent à freiner le déplacement du panneau 2 et peuvent à la limite conduire à son blocage. D'autre part, les diminutions de l'épaisseur $e$ peuvent conduire à une instabilité du panneau 2 dans des directions perpendiculaires à son plan.

La majeure partie de ces défauts est évitée, grâce notamment à la souplesse des rouleaux d'extrémité 44, 45. Néanmoins, de faibles diminutions de l'épaisseur $e$, bien qu'étant absorbées par le rouleau d'extrémité 44,45, notamment par la partie de la surface portante 68 située en vis à vis du bord plat 9, tendent à diminuer la poussée exercée par le rouleau d'extrémité 44,45 sur le panneau 2 qui n'est plus alors maintenu dans les meilleures conditions ; ceci tend à diminuer la qualité du déplacement du panneau 2, et à procurer une sensation désagréable d'instabilité lors de sa manipulation. Dans l'invention, pour éviter ces effets, le rouleau d'extrémité 44,45 comporte sur sa surface portante 68, une lèvre 71 formant une épaisseur additionnelle qui tend à s'écraser plus ou moins en fonction des variations d'épaisseur du bord plat 9 ; cette lèvre 71 étant en appui sur le bord plat 9.

La figure 4 montre une partie du premier rouleau d'extrémité 44, située dans un encadré 72 représenté sur la figure 1 et contenant la lèvre 71, qui peut ainsi être représenté plus en détail sur la figure 4.

Dans l'exemple non limitatif décrit, la lèvre 71 prolonge la surface portante 68 vers le côté 70 et comporte une seconde épaisseur e1 formant une épaisseur additionnelle progressivement croissante. Dans l'exemple non limitatif décrit, la lèvre 71 est réalisée dans un même matériau que le support d'extrémité 44, de manière à être obtenue par exemple de construction à la réalisation du rouleau d'extrémité 44. La lèvre 71 peut être comprise dans la longueur de la surface portante 68, où ainsi que dans l'exemple non limitatif décrit elle peut constituer une longueur additionnelle L1, de manière à former sur le côté 70 du premier rouleau 44 une pièce additionnelle 75 dont une troisième épaisseur e3, considérée selon un plan perpendiculaire au plan du panneau, permet de mieux obtenir la souplesse désirée de la lèvre 71.

Il est indiqué, à titre d'exemple non limitatif, que pour un bord plat 9 dont la dimension parallèlement au premier axe 10 est de l'ordre de 20 mm, la lèvre 71 comporte une longueur additionnelle de l'ordre de 10 mm et une épaisseur e1 de l'ordre de 1 mm, et que la troisième épaisseur e3 est de l'ordre de 10 mm ; la longueur L2 de la surface portante 68 étant sensiblement de 70 mm.

Cette description constitue un exemple non limitatif, montrant comment utiliser un arbre moteur pour supporter des éléments normalement indépendants de cet arbre moteur, en vue d'éviter la multiplication d'axes supports pour supporter ces éléments, et permettre ainsi la réalisation d'une table d'examen plus compacte et à moindre coût que dans l'art antérieur.

**Revendications**

1. Table d'examen comprenant:
 – un panneau (2) à déplacement suivant un axe longitudinal,
 – un socle fixe (4),
 – un arbre moteur (26), fixé audit socle (4) et tournant autour d'un premier axe (10) perpendiculaire à l'axe longitudinal dudit panneau (2),
 – des premiers moyens (54, 55) pour entraîner en rotation ledit arbre moteur (26),
 – au moins un premier rouleau (14, 44, 45) monté sur ledit arbre moteur (26) pour supporter ledit panneau (2),

 – au moins un second rouleau (12) fixé audit socle (4) et tournant autour d'un second axe (13) perpendiculaire à l'axe longitudinal dudit panneau (2) pour supporter ledit panneau (2) à une certaine distance dudit premier rouleau (10),
 – au moins un troisième rouleau (31 ou 32) tournant autour d'un troisième axe perpendiculaire à l'axe longitudinal dudit panneau (2), ledit troisième axe étant disposé à proximité du premier axe (10) et ledit troisième rouleau (31, 32) étant en contact avec une surface de roulement dudit panneau (2) de manière à exercer une force de réaction vis-à-vis du premier rouleau (14, 44, 45),
 – au moins un quatrième rouleau (35) fixé audit socle (4) et tournant autour d'un quatrième axe perpendiculaire à l'axe longitudinal dudit panneau (2), ledit quatrième axe étant disposé à proximité dudit second axe (13) et ledit quatrième rouleau (35) étant en contact avec ladite surface de roulement dudit panneau (2) de manière à exercer une force de réaction vis-à-vis dudit second rouleau (12),
 – des seconds moyens (16, 23, 25, 27, 19) pour convertir le mouvement circulaire de rotation dudit arbre moteur (26) en un mouvement linéaire dudit panneau (2) parallèlement audit axe longitudinal, caractérisé en ce que ledit premier rouleau (14, 44, 45) est monté sur ledit arbre moteur (26) de manière à tourner indépendamment dudit arbre moteur par l'intermédiaire d'un roulement (47, 49), et en ce que ledit troisième rouleau (31 ou 32) est monté sur un bras (33 ou 34) monté sur ledit arbre moteur (26) par l'intermédiaire d'un roulement (50).

2. Table d'examen selon la revendication 1, caractérisée en ce que lesdits seconds moyens comprennent:
 – au moins un pignon cranté (25) monté solidaire à une extrémité de l'arbre moteur (26), et
 – une courroie (16) coopérant avec ledit pignon cranté et ayant une première extrémité attachée en un premier point dudit panneau (2) et une deuxième extrémité attachée en un deuxième point dudit panneau (2) opposé au premier point par rapport audit premier axe (10).

3. Table d'examen selon la revendication 2, caractérisée en ce que lesdits seconds moyens comprennent en outre une première et une seconde poulies (23, 27) tournant, respectivement, autour d'un cinquième et sixième axes perpendiculaires à l'axe longitudinal dudit panneau (2) et disposées de part et d'autre dudit axe longitudinal de manière à recevoir ladite courroie (16).

**Claims**

1. An examination table comprising:
 – a panel (2) adapted to be displaced along a longitudinal axis,
 – a fixed base (4),
 – a drive arbor (26), secured to the said base (4) and turning about a first axis (10) perpendicular to the longitudinal axis of the said panel (2),
 – first means (54 and 55) in order to cause rotation of the said drive arbor (26),

– at least one first roll (14, 44 and 45) mounted on the said drive arbor (26) in order to support the said panel (2),

– at least one second roll (12) secured to the said base (4) and turning about a second axis (13) perpendicular to the longitudinal axis of the said panel (2) in order to support the said panel (2) at a certain distance from the said first roll (10),

– at least one third roll (31 or 32) rotating about a third axis perpendicular to the longitudinal axis of the said panel (2), the said third axis being located near the first axis (10) and the said third roll (31 and 32) being in contact with a roll surface of the said panel (2) in such a manner as to exert a reaction force in relation to the first roll (14, 44 and 45),

at least one fourth roll (35) secured to the said base (4) and rotating about a fourth axis perpendicular to the longitudinal axis of the said panel (2), the said fourth axis being located near the said second axis (13) and the said fourth roll (35) being in contact with the said roll surface of the said panel (2) in such a manner as to exert a reaction force in relation to the second roll (12),

– second means (16, 23, 25, 27 and 19) in order to convert the circular motion of rotation of the said drive arbor (26) into a linear motion of the said panel (2) in parallelism to the said longitudinal axis, characterized in that the said first roll (14, 44 and 45) is mounted on the said drive arbor (26) in such a manner as to turn independently of the said arbor by the intermediary of an anti-friction bearing (47 and 49), and in that the said third roll (31 or 32) is mounted on an arm (33 or 34) mounted on the said drive shaft (26) by the intermediary of an anti-friction bearing (50).

2. The examination table as claimed in claim 1 characterized in that the said second means comprise:

– at least one pinion (25) mounted in a fixed manner on one end of the drive arbor (26), and

– a belt cooperating with the said pinion (25) and having a first end attached at a first point of the said panel (2) and a second end attached at a second point of the said panel (2) opposite to the first point in relation to the said first axis (10).

3. The examination table as claimed in claim 2, characterized in that the said second means furthermore comprise a first and a second pulley (23 and 27) respectively turning about a fifth and a sixth axis perpendicular to the longitudinal axis of the said panel (2) and placed on the two sides of the said longitudinal axis in such a manner as to take up the said belt (16).

## Patentansprüche

1. Untersuchungstisch mit:
– einer Patientenlagerstatt (2), die entlang einer Längsachse verfahrbar ist,
– einem festen Sockel (4),
– einer Antriebswelle (26), die an diesem Sockel (4) befestigt ist und sich und um eine erste Achse (10) dreht, die senkrecht zur Längsachse dieser Patientenlagerstatt (2) verläuft,

– ersten Mitteln (54, 55), um diese Antriebswelle (26) in Drehung zu versetzen,
– mindestens einer ersten Rolle (14, 44, 45), die auf diese Antriebswelle (26) montiert ist, um diese Patientenlagerstatt (2) zu stützen,
– mindestens einer zweiten Rolle (12), die auf diesem Sockel (4) befestigt ist und sich um eine zweite Achse (13) dreht, die senkrecht zur Längsachse dieser Patientenlagerstatt (2) verläuft, um diese Patientenlagerstatt (2) in einem gewissen Abstand von dieser ersten Rolle (10) abzustützen,
– mindestens einer dritten Rolle (31 oder 32), die sich um eine dritte Achse dreht, die senkrecht zur Längsachse dieser Patientenlagerstatt (2) verläuft, wobei diese dritte Achse in der Nähe der ersten Achse (10) angeordnet ist und diese dritte Rolle (31, 32) mit einer Lauffläche dieser Patientenlagerstatt (2) in Kontakt ist, um so eine Reaktionskraft gegenüber der ersten Rolle (14, 44, 45) auszuüben,
– mindestens einer vierten Rolle (35), die an diesem Sockel (4) befestigt ist und sich um eine vierte Achse dreht, die senkrecht zur Längsachse dieser Patientenlagerstatt (2) verläuft, wobei diese vierte Achse in der Nähe dieser zweiten Achse (13) verläuft und diese vierte Rolle (35) in Kontakt mit dieser Lauffläche dieser Patientenlagerstatt (2) ist, um so eine Reaktionskraft gegenüber dieser zweiten Rolle (12) auszuüben,
– zweiten Mitteln (16, 23, 25, 27, 19), um die kreisförmige Rotationsbewegung dieser Antriebswelle (26) in eine Linearbewegung dieser Patientenlagerstatt (2) parallel zu dieser Längsachse umzuwandeln,
dadurch gekennzeichnet, daß diese erste Rolle (14, 44, 45) auf dieser Antriebswelle (26) so montiert ist, daß sie sich unabhängig von dieser Antriebswelle mittels eines Rollenlagers (47, 49) dreht, und dadurch, daß diese dritte Rolle (31 oder 32) auf einem Arm (33 oder 34) angebracht ist, der auf dieser Antriebswelle (26) mittels eines Rollenlagers (50) montiert ist.

2. Untersuchungstisch nach Anspruch 1, dadurch gekennzeichnet, daß diese zweiten Mittel umfassen:
– mindestens eine Zahnscheibe (25), die fest an ein Ende der Antriebswelle (26) montiert ist, und
– einen Riemen (16), der mit dieser Zahnscheibe zusammenwirkt und ein erstes Ende hat, das in einem ersten Punkt dieser Patientenlagerstatt (2) angebracht ist, und ein zweites Ende, das in einem zweiten Punkt dieser Patientenlagerstatt (2) angebracht ist, der bezüglich dieser ersten Achse (10) entgegengesetzt zum ersten Punkt liegt.

3. Untersuchungstisch nach Anspruch 2, dadurch gekennzeichnet, daß diese zweiten Mittel außerdem eine erste und eine zweite Riemenscheibe (23, 27) umfassen, die sich um eine fünfte bzw. sechste Achse drehen, die senkrecht zur Längsachse dieser Patientenlagerstatt (2) verlaufen und beiderseits dieser Längsachse angeordnet sind, um diesen Riemen (16) aufzunehmen.

FIG_1

# FIG_2

EP 0 226 515 B1

# FIG_3

# FIG_4